Europäisches Patentamt

European Patent Office

Office européen des brevets

(19)

(11) Numéro de publication: **0 300 926**
**A1**

(12) **DEMANDE DE BREVET EUROPEEN**

(21) Numéro de dépôt: **88420134.4**

(22) Date de dépôt: **22.04.88**

(51) Int. Cl.⁴: **A 61 F 2/24**

(30) Priorité: **29.04.87 FR 8706347**

(43) Date de publication de la demande:
**25.01.89 Bulletin 89/04**

(84) Etats contractants désignés:
**AT BE CH DE ES GB GR IT LI LU NL SE**

(71) Demandeur: **Cuilleron, Jean**
**57, rue Francisque Voytier**
**F-42100 Saint-Etienne (FR)**

**Dureau, Georges**
**6 rue de la Mairie**
**F-69340 Francheville (FR)**

**SOCIETE CERIC CENTRE D'ETUDES ET DE RECHERCHE**
**DE L'IMPLANT CHIRURGICAL, SARL**
**"Les Algorithmes" E2 route des Lucioles Sophia**
**Antipolis**
**F-06500 Valbonne (FR)**

(72) Inventeur: **Dureau, Georges**
**6, rue de la Mairie**
**69340 Francheville (FR)**

(74) Mandataire: **Dupuis, François**
**Cabinet Charras 3 Place de l'Hôtel-de-Ville BP 203**
**F-42005 St. Etienne Cédex 1 (FR)**

(54) **Valve cardiaque.**

(57) Cette valve est du type comprenant un moyen s'insérant avec un débattement contrôlé et limité dans une cage (2) comprenant un anneau (2.1) et une ossature en fil constituant des arceaux, ledit anneau présentant sur sa périphérie extérieure une rainure périphérique (2.2) autorisant le centrage et le positionnement d'un habillage (3), ladite valve étant caractérisée en ce que ledit moyen s'insérant dans la cage est un module comprenant une demi-sphère (1.1) prolongée à l'avant par un doigt (1.3) débouchant de l'anneau vers l'extérieur, ledit anneau présentant un profil intérieur (2.4) formant siège autorisant le centrage et le basculement angulaire et l'articulation du module (1) en toute position de balayage du doigt.

FIG.1

EP 0 300 926 A1

Bundesdruckerei Berlin

## Description

### Valve cardiaque.

La présente invention a pour objet une valve cardiaque.

On connaît sur le marché différents types de valves, types valves à clapets ou à disques, valves à bille.

Ces valves présentent un inconvénient dans le fait qu'elles peuvent être obturées progressivement par l'apparition de fibrine se formant sur et autour de l'anneau ou armature métallique constituant le siège de la valve.

On connait également par le brevet US 3.601.877 une valve comprenant un module en deux parties associées se prolongeant par des doigts en saillie, servant au guidage du module dans un mouvement de va et vient centré dans des anneaux de l'armature support de la valve. Ce type de valve fonctionne à la manière d'un piston sans répondre au problème posé du dépôt de fibrine.

D'autres publications ont décrits l'utilisation de doigt de prolongement du module de la valve dans une fonction de guidage de mouvement linéaire, tels par exemple les brevets 1.398.325, AU 441906, US 3503079, sans pour autant répondre au problème posé.

Il a même été suggéré une solution originale par le Brevet FR 1449117 pour tenter de trouver une solution à l'apparition de fibrine eu égard notamment aux valves cardiaques comportant une armature ou boîte à cage. Cette solution consiste à réaliser un anneau en un matériau particulier et de l'implanter lors d'une première opération chirurgicale sur le patient. Cette implantation permet la formation sur l'anneau d'un tissu de façon à obtenir un anneau revitalisé. Celui-ci est ensuite réimplanté lors d'une seconde opération chirurgicale dans le coeur en y adjoignant la valve. Celle-ci est montée libre en étant maintenue et guidée par deux doigts orientés angulairement et constituant des limiteurs de course et de butée. Selon le titu laire de ce brevet, la revitalisation préalable de l'anneau est de nature en elle-même à empêcher la formation de la fibrine.

En pratique, et à la connaissance des demandeurs, un tel concept n'est pas exploité en égard peut être et en particulier aux exigences de sécurité de la valve cardiaque ainsi conçue tenant à la seule fiabilité des deux ergots dans des conditions d'utilisation normales de battement du coeur.

Le but recherché selon l'invention était de remédier au problème posé d'apparition de la fibrine et d'assurer la fiabilité de fonctionnement de la valve dans le temps.

On a voulu améliorer la qualité de fonctionnement de celles-ci, soumises à un nombre considérable de mouvements dans le temps, et dans un milieu particulier, pour permettre un meilleur écoulement du sang et éviter toutes formations de thromboses dans l'environnement immédiat des valves.

Selon une première caractéristique, la valve cardiaque est du type comprenant un moyen s'insérant avec un débattement contrôlé et limité dans une cage comprenant un anneau et une ossature en fil constituant des arceaux, ledit anneau présentant sur sa périphérie extérieure une rainure périphérique autorisant le centrage et le positionnement d'un habillage, ledit moyen présentant dans le prolongement de son plan axial un doigt en forme de saillie; ladite valve étant remarquable en ce que ledit moyen s'insérant dans la cage est un module comprenant une demi-sphère prolongée à l'avant par un doigt débouchant de l'anneau vers l'extérieur, ledit anneau présentant un profil intérieur sphérique formant siège autorisant le centrage et le basculement angulaire et l'articulation du module en toute position de balayage du doigt, assurant un auto-nettoyage des périphéries interne et externe de l'anneau formant siège.

Ces caractéristiques et d'autres encore ressortiront bien de la suite de la description.

Pour fixer l'objet de l'invention illustrée d'une manière non limitative aux figures des dessins où :

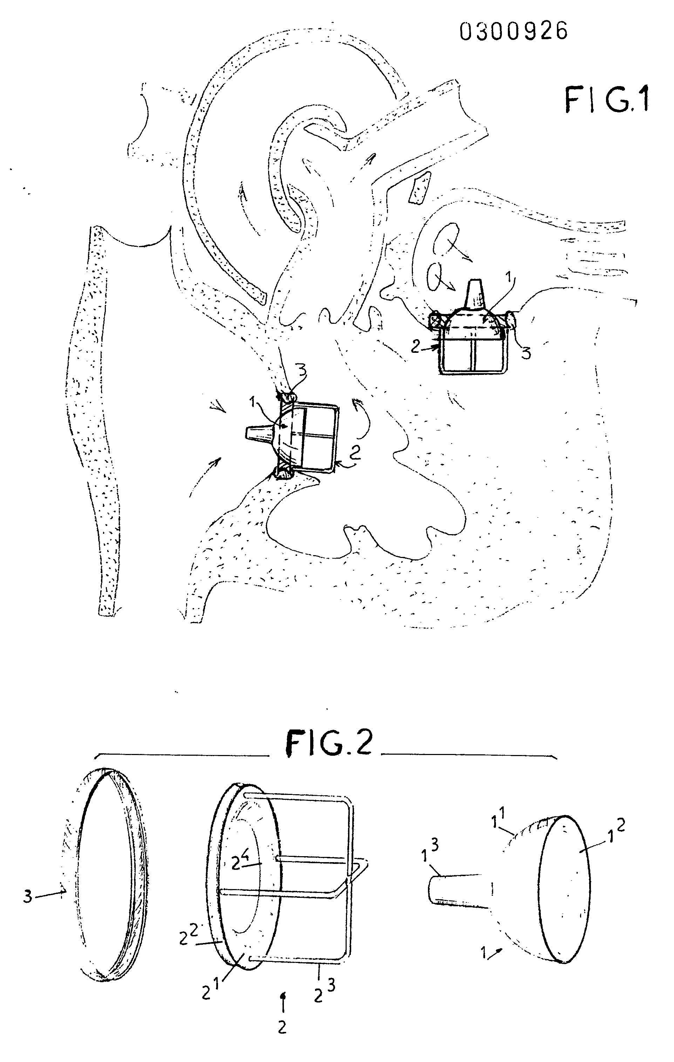

La figure 1 est une vue en coupe d'un coeur humain illustrant le positionnement, selon deux positions possibles et non limitatives, de la valve selon l'invention.

La figure 2 est une vue en perspective éclatée des différents éléments constitutifs de la valve.

Les figures 3, 4, 5 et 6 sont des vues à caractère schématique illustrant les différentes positions du module par rapport à la cage de réception.

Afin de montrer plus concrètement l'objet de l'invention, on le décrit maintenant d'une manière non limitative illustrée aux figures des dessins.

La valve comprend un moyen établi sous forme de module (1) s'insérant avec un débattement contrôlé et limité dans une cage (2). Celle-ci comprend un anneau (2.1) profilé présentant une rainure périphérique (2.2) extérieure autorisant le centrage et le positionnement d'un habillage (3) réalisé en tous matériaux appropriés, permettant sa suture sur les lèvres en regard des parois de l'orifice de communication entre par exemple l'oreillette et le ventricule. Ledit anneau (2.1) se prolonge à l'arrière pour former cage avec une ossature en fil (2.3) définie pour comprendre trois ou quatre branches en U à fond plat de préférence constituant des arceaux. La cage ainsi réalisée est monobloc et en tous matériaux appropriés, avec ou sans points de liaison ou de jonction entre les arceaux et l'anneau.

Selon l'invention, ladite cage reçoit un module (1) comprenant une demi-sphère (1.1) à plan équatorial plat (1.2) et prolongée vers l'avant dans son plan axial du côté bombé par un doigt (1.3) ayant de préférence un profil en tronc de cône, pour un meilleur écoulement du sang. A titre complémentaire, la partie intérieure périphérique de l'anneau (2.1) est agencée avec un profil complémentaire sphérique (2.4), par exemple et non limitativement, formant siège autorisant le centrage et le basculement angulaire et l'articulation du module (1) par sa partie semi-sphérique (1.1) d'appui.

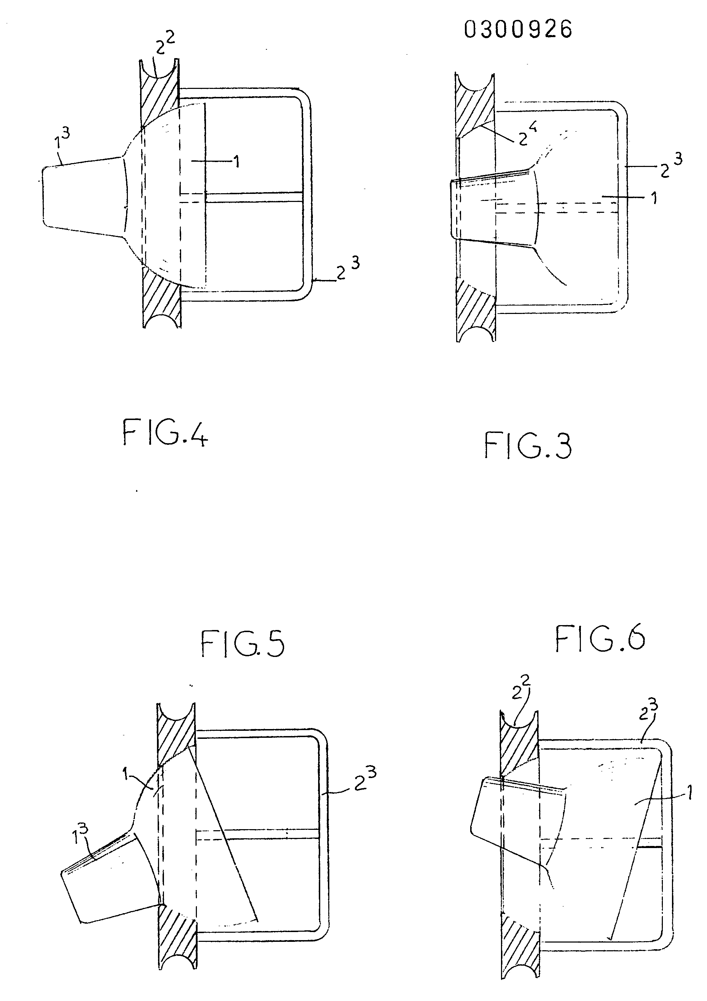

Ainsi qu'il apparaît aux dessins, en position ouverte de la valve (figure 3), c'est-à-dire autorisant le passage du sang, le module est plaqué contre le fond de la cage, tandis que le doigt (1.3) obture de manière minimum l'ouverture à accès par l'anneau ; l'extrémité avant du doigt vient sensiblement dans le plan transversal extérieur de l'anneau.

En position fermée (figure 4), le module prend appui sur le siège (2.4) de l'anneau en occluant le passage et évitant toute circulation du sang, le doigt (1.1) débordant vers l'extérieur. La suppression de la partie supérieure de la sphère dégage le volume intra-ventriculaire et notamment la chambre de chasse aortique.

En se référant aux figures 5 et 6, le module se situe en position oblique dans la cage. La position illustrée figure 5 à l'occlusion limitée par le doigt (1.3) en forme de tronc de cône est la plus fréquente du fait de l'asymétrie du module et du poids du tronc de cône ou doigt. Ce dernier dégage complètement la chambre de chasse aortique. Le doigt (1.3) vient en contact avec la périphérie interne (2.5) de l'anneau en venant racler ou araser le long de celle-ci, tandis que la partie semi-sphérique est en étroite liaison avec le profil (2.4) intérieur de l'anneau. La périphérie externe (2.6) de l'anneau est auto-nettoyée par la partie semi-sphérique du module. La position illustrée figure 6 du module en position oblique à l'ouverture rend impossible la luxation car le doigt (1.3) vient toujours en butée contre la périphérie interne de l'anneau. Cette position est généralement instantanément suivie par un positionnement correspondant à la figure 3. Il y a lieu d'observer que le module par sa forme ne peut plus être dégagé de la cage une fois mis en place par tous procédés techniques appropriés.

Une des fonctions nouvelles de cette valve consiste à permettre par les multiples positions du doigt, un balayage complet de tout l'orifice de la valve du côté atrial s'opposant à la formation de panus qui peut limiter l'orifice valvulaire. Ce panus résulte de pousses cellulaires susceptibles de se former autour de l'anneau à partir de la zône de suture de l'habillage et gêner ainsi la circulation du sang par obturation progressive de l'orifice de passage. Le balayage continu donné par le mouvement du doigt du module permet ainsi d'obtenir un orifice d'ou verture constante et exempte de tous corps non désirés. Cette fonction nouvelle du module est particulièrement importante et constitue une indéniable amélioration dans le fonctionnement des valves dans le temps. En effet, selon l'art antérieur, les valves à clapets, à disques ou à billes, ne présentent aucun moyen susceptible de déborder de l'anneau récepteur et assurer un autoentretien. Les valves avec doigt telles que rappelées dans le préambule de la demande n'ont qu'une fonction de centrage en position par un mouvement linéaire en va et vient.

Le module selon l'invention, ainsi que les autres éléments de la valve peut être avantageusement et non limitativement réalisé en carbone pyrolitique. La densité du module doit être égale à celle du sang.

Par ailleurs la cage de la valve selon l'invention peut être d'une hauteur plus réduite par rapport par exemple à une valve à billes et donc d'un encombrement moindre, tout en autorisant un déplacement du module (1) plus importante que celle-ci. Ce déplacement supplémentaire permet une amélioration de l'écoulement au travers de l'anneau compensant la perte de surface du doigt au niveau de l'ouverture de l'orifice valvulaire en position ouverte.

Les avantages ressortent bien de l'invention. Ou souligne :

- la fiabilité d'utilisation de la valve,
- l'absence de formation de thrombose fréquemment rencontrée au niveau des articulations pour les valves à clapet,
- le dégagement de la chambre de chasse aortique lorsque la valve est utilisée en position mitrale la plus fréquente,
- le balayage constant de l'orifice valvulaire du côté atrial s'opposant à la formation de panus.

Les valves ainsi réalisées selon l'invention peuvent se faire, pour chacun des différents éléments constitutifs, en toutes dimensions et matériaux appropriés.

## Revendications

-1- Valve cardiaque du type comprenant un moyen s'insérant avec un débattement contrôlé et limité dans une cage (2) comprenant un anneau (2.1) et une ossature en fil constituant des arceaux, ledit anneau présentant sur sa périphérie extérieure une rainure périphérique (2.2) autorisant le centrage et le positionnement d'un habillage (3), ledit moyen présentant dans le prolongement de son plan axial un doigt en forme de saillie, ladite valve étant caractérisée en ce que ledit moyen s'insérant dans la cage est un module comprenant une demi-sphère (1.1) prolongée à l'avant par un doigt (1.3) débouchant de l'anneau vers l'extérieur, ledit anneau présentant un profil intérieur (2.4) formant siège autorisant le centrage et le basculement angulaire et l'articulation du module (1) en toute position de balayage du doigt, assurant un auto-nettoyage des périphéries interne (2.5) et externe (2.6) de l'anneau formant siège.

-2- Valve cardiaque selon la revendication 1, caractérisée en ce que le doigt (1.3) présente une forme en tronc de cône.

-3- Valve cardiaque selon la revendication 1, caractérisée en ce que, en position ouverte de la valve, le module est plaqué contre le fond de la cage et que l'extrémité avant du doigt vient sensiblement dans le plan transversal extérieur de l'anneau.

-4- Valve cardiaque selon la revendication 1, caractérisée en ce que le profil intérieur (2.4) de l'anneau est sphérique.

-5- Valve cardiaque selon les revendications 1 et 3 ensemble, caractérisée en ce que le module (1) comprend une demi-sphère (1.1) à plan équatorial plat (1.2.).

0300926

FIG.1

FIG.2

0300926

FIG.4

FIG.3

FIG.5

FIG.6

| | | |
|---|---|---|
| ((( Office européen des brevets | RAPPORT DE RECHERCHE EUROPEENNE | Numero de la demande |

EP 88 42 0134

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-3 601 877 (C.C. GOOSEN)<br>* Figures 1,2; colonne 2, lignes 15-30 * | 1,4 | A 61 F 2/24 |
| | --- | | |
| X | FR-A-1 449 117 (J. UHLIR et al.)<br>* Figures 1,2 * | 1,3 | |
| | --- | | |
| A | US-A-3 503 079 (C.A. SMITH)<br>* Figures 1,2,5 * | 1-5 | |
| | --- | | |
| A | FR-A-1 398 325 (J. ALTMAN)<br>* Figure 3 * | 1-5 | |
| | --- | | |
| A | AU-B- 452 280 (UNIVERSITY OF MINNESOTA)<br>* Figure 14 * | 1-5 | |
| | ----- | | |

**DOMAINES TECHNIQUES RECHERCHES (Int. Cl.4)**

A 61 F

**Le présent rapport a été établi pour toutes les revendications**

| Lieu de la recherche | Date d'achèvement de la recherche | Examinateur |
|---|---|---|
| LA HAYE | 14-07-1988 | ARGENTINI A. |